# EUROPEAN PATENT SPECIFICATION

(11) **EP 2 419 176 B1**
(45) Date of publication and mention of the grant of the patent: **14.05.2014**
(21) Application number: 10713974.3
(22) Date of filing: 16.04.2010
(51) Int. Cl.: A61K 8/31, A61P 17/16, A61K 31/01, A61K 31/047, A61K 45/06, A61K 9/06, A61Q 19/00, A61K 47/06, A61K 47/10, A61K 9/10, A61K 9/107, A61K 8/34

(54) **PHARMACEUTICAL COMPOSITION COMPRISING GLYCEROL, WHITE SOFT PARAFFIN AND LIQUID PARAFFIN FOR USE IN THE TREATMENT OF UREMIC PRURITUS**
GLYCEROL, WEISSES WEICHES PARAFFIN UND FLÜSSIGES PARAFFIN ENTHALTENDE PHARMAZEUTISCHE ZUSAMMENSETZUNG ZUR VERWENDUNG IN DER BEHANDLUNG VON URÄMISCHES PRURITUS
COMPOSITION PHARMACEUTIQUE COMPRENANT DU GLYCÉROL, DE LA PARAFFINE MOLLE BLANCHE ET DE LA PARAFFINE LIQUIDE POUR UTILISATION DANS LE TRAITEMENT D'UN PRURIT URÉMIQUE

(30) Priority: 16.04.2009 EP 09305325; 01.09.2009 US 272207 P
(43) Date of publication of application: 22.02.2012
(73) Proprietor: Orfagen, 31520 Ramonville Saint Agne (FR)
(72) Inventor: DUPUY, Patrick, F-31000 Toulouse (FR)
(74) Representative: Regimbeau
(86) International application number: PCT/EP2010/055066
(87) International publication number: WO 2010/119132

(56) References cited:
- LABORATOIRES PIERRE FABRE: "Rapport annuel 2005" , [Online] 2005, page 19, XP002540897 Retrieved from the Internet: URL:http://www.pierre-fabre.com/file/11514 18610843_pf_rapport2005.pdf> [retrieved on 2009-08-11]
- ANONYMOUS: "DEXERYL, Notice d'utilisation" INTERNET CITATION, [Online] 14 June 2007 (2007-06-14), XP002540898 Retrieved from the Internet: URL:http://www.pierrefabre-dermatologie.de /produkte/dexeryl/beipackzettel-def-dexery l-2007/at_download/file> [retrieved on 2009-08-11]
- FAIVRE B ET AL: "Étude de l'action de Dexeryl(R) versus dexpanthénol sur la xérose et la desquamation: évaluation par cornéométrie et méthode D-Squames(R)" NOUVELLES DERMATOLOGIQUES 200701 FR, vol. 26, no. 1, January 2007 (2007-01), pages 42-45, XP009121325 ISSN: 0752-5370
- ANONYMOUS: "Dexeryl crème" VIDAL, [Online] 30 June 2004 (2004-06-30), XP002540899 Retrieved from the Internet: URL:http://web.archive.org/web/20040630050 212/http://www.vidal.fr/Medicament/dexeryl -5021.htm> [retrieved on 2009-08-11]
- ANONYMOUS: "New emollient cream Dexeryl (R) preferred by patients with ichthyosis and eczema" MEDICAL NEWS TODAY, [Online] 20 August 2008 (2008-08-20), XP002540900 Retrieved from the Internet: URL:http://www.medicalnewstoday.com/articl es/118718.php> [retrieved on 2009-08-11]
- SZEPIETOWSKI JACEK C ET AL: "Uraemic xerosis" NEPHROLOGY DIALYSIS TRANSPLANTATION, vol. 19, no. 11, November 2004 (2004-11), pages 2709-2712, XP002540901 ISSN: 0931-0509 cited in the application
- OKADA KAZUYOSHI ET AL: "Effect of skin care with an emollient containing a high water content on mild uremic pruritus." THERAPEUTIC APHERESIS AND DIALYSIS : OFFICIAL PEER-REVIEWED JOURNAL OF THE INTERNATIONAL SOCIETY FOR APHERESIS, THE JAPANESE SOCIETY FOR APHERESIS, THE JAPANESE SOCIETY FOR DIALYSIS THERAPY OCT 2004, vol. 8, no. 5, October 2004 (2004-10), pages 419-422, XP002540902 ISSN: 1744-9979
- MORTON C A ET AL: "Pruritus and skin hydration during dialysis" NEPHROLOGY DIALYSIS TRANSPLANTATION, vol. 11, no. 10, 1996, pages 2031-2036, XP002581849 ISSN: 0931-0509
- KEITHI-REDDY S R ET AL: "Uremic pruritus" KIDNEY INTERNATIONAL, vol. 72, no. 3, August 2007 (2007-08), pages 373-377, XP002581850 ISSN: 0085-2538
- KUYPERS DIRK R J: "Skin problems in chronic kidney disease." NATURE CLINICAL PRACTICE. NEPHROLOGY MAR 2009 LNKD- PUBMED:19190625, vol. 5, no. 3, March 2009 (2009-03), pages 157-170, XP002588032 ISSN: 1745-8331

## Description

The invention relates to the treatment of uremic pruritus.

Patients undergoing maintenance renal dialysis (MRD) present with several dermatologic complications. Among these complications, xerosis (rough and scaly skin, also called uremic xerosis) and pruritus (also called uremic pruritus) are the most common manifestations. Both of these non specific symptoms are also observed in patients with chronic renal failure before the need of dialysis, but are significantly increased when patients initiate MRD, either haemodialysis or peritoneal dialysis. They are classically absent in acute renal failure, and are not correlated with the plasmatic levels of urea. Furthermore, they typically disappear after renal transplantation. Uremic xerosis is a condition that is clearly delineated from the other xerotic conditions (common xerosis, atopic xerosis, etc.) by its clinical signs, pathogenesis and therapeutic management.

Uremic xerosis is a very common complaint in patients who undertake MRD. According to the literature, it is affecting 50% to 85% of the patients whereas 30 to 40% of patients report this symptom before starting MRD or after renal transplant. Age is an aggravating factor. It often affects all the body, and is usually more severe in some areas (legs, forearms, hands, back). In large series, the intensity of xerosis has been described as mild in 30-40%, moderate in 35-50%, and severe in 15-30% of the MRD patients. It is a chronic syndrome, with a clinical picture comprising a dry skin appearance, marked scaling and roughness, and a poor turgor (i.e. failure to the skin to reassume prompt normal contour, once the skin has been pulled upward) of the skin. Icthyosiform lesions with large scales and cracks as well as keratolytic pits or keratoderma on the palms and soles may be seen in the severe cases, featuring the dyskeratotic component of the condition. The negative influence of climate and environmental factors (wind, cold, low humidity) is frequently reported. Associated signs are premature skin ageing (elastosis), diminished sweating and pruritus (also called uremic pruritus).

Uremic xerosis is considered to be an important factor contributing to uremic pruritus. Like in xerosis, pruritus frequency in MRD patients is greatly increased, ranging from 35% to 65%, whereas it is observed in about 30% of patients with chronic renal failure without MRD. The frequency of uremic pruritus is higher in patients with xerosis than patients without. Based on large published series, xerosis of moderate to severe intensity leads to a 50-100% increase in uremic pruritus. Similarly, uremic pruritus severity appears to be directly related to xerosis severity : the more intense xerosis is, the greater the intensity of pruritus is.

By contrast, it is not significantly influenced by factors such as patient age, sex, underlying renal disease or type of dialyzate used for haemodialysis (acetate-based or bicarbonate-based), except for haemodialyzed patients using less permeable dialysis membranes (polysulphone), who may have a higher incidence of pruritus than patients dialyzed with more permeable (hemophane or cuprophane) membranes. Like uremic xerosis, uremic pruritus is a chronic symptom with, sometimes, periods of exacerbation during or soon after haemodialysis. It may be generalized or localized. Especially when associated with xerosis, pruritus provokes vigorous scratching, which can produce extensive excoriations, lichenification, prurigo nodularis (multiple brown nodules) and sometimes cutaneous superinfections (secondary impetigo). Bothersome disturbance of sleeping and daily activities are common. The pathophysiology of uremic pruritus is not understood and appears multifactorial. Among likely factors, uremic xerosis has been considered as a main factor.

The cause of uremic xerosis in MRD patients is unclear. Multiple factors may contribute towards the rough and scaly skin appearance of MRD patients, including skin dehydration and reduced sebum and sweat excretion. Another possible explanation is an alteration in the metabolism of vitamin A, which is in increased concentration in uremic patients. Xerotic skin of hypervitaminosis A syndrome resembles the skin lesions of xerotic patients with MRD. Atrophy of sebaceous glands, resulting in lower levels of surface lipids of the skin, may also participate in the pathogenesis of uremic xerosis. Other potential factors include thyroid underactivity and skin inflammation by the increased number of cutaneous mast cells releasing histamine.

The primary measure for treatment of uremic xerosis in MRD patients remains the generous use of topical emollients. Demonstration of its efficacy has never been shown but is universally accepted by therapists. Moisturizing emollients have either mainly rehydrating effects or occlusive properties, and optimal effects are reached when used on a regular basis, i.e. at least twice a day in a continuous course. However, it is also known that patients with uremic xerosis are resistant to conventional emollients, especially severe cases, and are particularly prone to develop irritancy to topical products as well as soaps and detergents.

One main problem for uremic xerosis patients is the poor outcome achieved on the lesions with available moisturizing emollients¹. This is especially true in more severe cases. Several hypotheses may be proposed to explain the poor response of the lesions to conventional emollience therapy: 1°) the continuous skin dehydration process that occurs during the repeated MRD sessions; 2°) the dyskeratotic component of the condition, which results in persistent barrier dysfunction and requires strong occlusive treatment. Similarly, emollient therapy has some beneficial effect on uremic pruritus but remains partial, with a marked relief of pruritus observed in 33 to 43% of the patients only using a twice daily application^{2,3}. As a result, no emollient product has been developed as medicinal product in uremic xerosis, and patients are bound to try by themselves numerous emollient products that are available on cosmetic market. The use of a great variety of emollients aggravates their intolerance to tensio-actives and other irritants contained in classical emollients⁴.

An additional problem with the currently available emollient products is that they comprise an unlimited number of non-active ingredients, including fragrance materials and tensio-actives at high concentration. This exposure to an excessive number of ingredients and an excessive dose is potentially a higher risk for MRD patients to develop local intolerance than it might be for healthy individuals.

Also, the problem with a treatment based on emollient products is the cost of emollients. This is possibly a factor of poor compliance for patients with MRD. Conditions of efficacy of an emollient include generous and repetitive applications of the product every day on xerotic areas, i.e. over a large area of the body surface. Treatment is long-lasting. Available products are registered under the cosmetic regulations in most countries, including the European Community. None of them has been approved in the proposed indication, and a few of them have been marketed as ethical products in other indications. Nonetheless, MRD patients have a medical need for these products, and they cannot be provided to them other than through the cosmetic market. Consequently, products are only available to patients at full cost. The material to apply should be large, about 1 mg/cm², to avoid incomplete coverage, resulting in high consumption of the product in normally compliant patients. For instance, this would require a consumption of about 250 ml/week in adult patients for total coverage (total body surface area (BSA) : 1.70 m², surface application : 50% BSA, 2mg/cm² to apply, 2 applications/day, product density ≈ 1000). Providing that the approximate cost of an emollient in the European cosmetic market is around 10 Euros for 100 ml of product, the expense could be estimated monthly up to 100 Euros for an adult. Without substantial reimbursement of the product, it is obvious that the effective cost of emollients is very likely to alter their optimum use by patients affected by the proposed indication.

Thus, there exist important needs for an emollient treatment method, approved as a medicinal product, showing good clinical efficacy in normalizing uremic pruritus, as well as better compliance and better tolerance in the target population.

For this purpose, the present inventors have shown that an emollient based on glycerol and paraffin disclosed essential characteristics to be potentially efficacious and safe in uremic xerosis patients, as follows: 1°) to exert a rapid hydrating effect, using glycerol at active dose (15%); 2°) to preserve the barrier function against irritants using paraffin combining both soft form and liquid form; and 3°) to accelerate the normalization of dyskeratosis and skin barrier repair, with the synergistic action of glycerol and paraffin.

Second, the present inventors performed two randomized, placebo-controlled, double-blind studies showing therapeutic efficacy of an emollient based on glycerol and paraffin on uremic xerosis. The two studies confirmed the good efficacy and excellent safety profile of the combination of the two active substances in a suitable oil-in-water (o/w) emulsion. In addition, it was showed unexpected good results on the reduction of uremic pruritus, i.e. a decrease by about 75% of the pruritus. In one study, it was further demonstrated that lesional remission of uremic xerosis and pruritus relief can be achieved with a once daily application of the product, whereas other moisturizing emollients are commonly used twice daily.

Patients undergoing MRD are advantageously concerned by a treatment of uremic xerosis according to the invention.

Therefore a first object of the invention relates to a pharmaceutical composition comprising a combination of glycerol, white soft paraffin and liquid paraffin as active ingredients for topical use to treat uremic pruritus.

Advantageously for decreasing side effects, said topical use has an once per day dosage regimen, preferably an once per day dosage regimen for at least 2 weeks, preferably 4 weeks.

Preferably, the composition for use according to the invention is a dermatological composition. Preferably, the composition for use according to the invention comprises less than 15, preferably 10, components.

Patients undergoing maintenance renal dialysis (MRD) are advantageously concerned by the treatment of uremic pruritus according to the invention.

Within the meaning of the present invention, « active association» means the association of glycerol, white soft paraffin and liquid paraffin.

Advantageously, white soft paraffin and liquid paraffin are controlled according to respectively monograph n°0496, Monograph n° 1799 and Monograph n°0239 of the « European Pharmacopeia », 6th Edition.

Advantageously, white soft paraffin of the active has a dropping point between 35 and 70°C, preferably between 51 and 57°C, more preferably of approximately 54°C. The dropping point is measured according to the process 2.2.17 described in « European Pharmacopeia », 6eme Edition.

Advantageously, white soft paraffin of the active association has a consistency between 175 and 195 1/10 mm, preferably about 185 1/10 mm (cone penetration at 25°C).

Advantageously, white soft paraffin of the active association has a viscosity between 4 and 5 cSt at 100°C, preferably about 4.8 cSt at 100°C.

The active association is present in the composition according to the invention, in a proportion between 10 and 50 % and preferably between 20 and 30 % w/w compared to the total weight of the composition ; the concentration of glycerol is comprised between 5 and 30 %, preferably between 10 and 20 % and more preferably is about 15% w/w compared to the total weight of the composition, the concentration of white soft paraffin is comprised between 3 and 20 %, preferably between 5 and 10 % and more preferably is about 8% w/w compared to the total weight of the composition and the concentration of liquid paraffin is comprised between 0.5 and 5 %, preferably between 1 and 3 % and more preferably is about 2% w/w compared to the total weight of the composition.

Water is comprised between 30 and 80 w/w compared to the total weight of the composition.

Advantageously, the composition according to the invention comprises about 15% of glycerol, about 8% of white soft paraffin and about 2% of liquid paraffin w/w compared to the total weight of the composition.

The dermatological composition according to the invention comprises furthermore usual dermatologically acceptable excipients.

Preferably, the dermatological composition according to the invention is a water-in-oil (W/O) or oil-in-water (O/W) emulsion, a water-in-oil-in-water (W/O/W) or oil-in-water-in-oil (O/W/O) emulsion, or a hydrodispersion or a lipodispersion, a gel or an aerosol, more preferably a water-in-oil (W/O) or a oil-in-water (O/W) emulsion, and the most preferably a oil-in-water (O/W) emulsion.

Indeed, the type of formulation (neat substances, solutions and emulsions) and the type of emulsion (W/O or O/W emulsion) intervene in the activity of the active association.

When used as neat substances, white soft paraffin had no occlusive activity when applied in powder form⁵, but a highly occlusive effect when applied in an oil phase (liquid state). This indicates that paraffin shows occlusive properties when applied uniformly on the skin surface. In order to apply white soft paraffin uniformly, a liquid state of white soft paraffin is required, e.g. as formulated in emulsions. Paraffin in emulsions is more occlusive than in solutions⁶.

The hydrating and occlusive effects of emulsions themselves vary according to their types (w/o and o/w). The w/o formulation with white soft paraffin even at high dosage (46.75%) is able to induce a significant increase in stratum corneum (SC) () water content, but has limited or no significant effect on skin barrier function by TransEpidermal Water Loss (TEWL) reduction ⁶. Similarly, o/w emulsions by themselves have a limited effect on skin hydration, or even deleterious effects on the SC barrier function ⁷. When associated with liquid paraffin, the o/w emulsion looses water faster than the w/o emulsion, and begins to show earlier but milder occlusivity than the w/o emulsion ⁵. By contrast, the adjunction of glycerol to paraffin in a*n* o/w formulation can prevent SLS (Sodium Laurylsulphate)-induced skin dehydration ⁷. Using the same SLS model, Grunewald and co-workers showed a similar synergistic effect between a o/w emulsion and glycerol as regards to prevention of skin dehydration, compared to urea in the same o/w formulation and other w/o commercial formulations ⁸. Glycerol in o/w emulsions acts through a long-term regenerative process of the disrupted SC, rather than a simple physicochemical mechanism of action such as hydration ^{9,10,11}.

In conclusion, on the one hand, o/w emulsions without glycerol have limited or transitory effects on the moisture of the SC a well as possible deleterious effects on the skin barrier, whereas the w/o emulsions exert a better hydration profile but a limited barrier effect. On the other hand, whereas w/o emulsions act merely as a protective film that induces skin hydration, o/w emulsions facilitate the hydrating and regenerative effects of glycerol.

Preferably, 90% of the paraffin droplets present in a composition according to the invention in the form of an emulsion have a size below 30 µm, more preferably 25 µm.

Indeed, the occlusive properties of white soft paraffin in emulsions also vary with the size of the droplets they constitute in the formulation ⁵. The smaller the paraffin particles are, the more occlusive white soft paraffin is, with an optimum threshold of activity being reached with droplet size under 30 µm. An internal stereomicroscopic study showed that more than 90% of the paraffin droplets dispersed in Composition A, Example 1, have a size below 25 µm. This indicates that the paraffin dispersion in the proposed formulation provides optimum bioavailability of this active compound.

Dermatologically acceptable excipients can be any excipient among those well-known by the one skilled in the art in order to obtain a composition for topical application in the form of a cream, a lotion, a gel, an ointment, an emulsion, a microemulsion, a spray, etc

The composition according to the invention can in particular comprise additives, such as appropriate emulsifying agents, consistency agents, gelling agents, water-binding agents, spreading agents, stabilizing agents, colouring agents, perfumes and preservatives.

Appropriate emulsifying agents comprise stearic acid, trolamine, lanolin alcohols (e.g. Amerchol L101, i.e. a mixture of 90% of mineral oil and 10% of lanolin alcoho ls).

Preferably, appropriate emulsifying agents do not have disruptive effects on the SC and, thus, do not modify the barrier function. The one skilled in the art will chose appropriate emulsifying agents according to his general knowledge. For example, stearic acid has been shown to induce no alteration on normal skin and SLS-modified skin in humans, at the concentration of 5% ¹².

Preferably, the composition according to the invention comprises about 5% of emulsifying agents w/w compared to the total weight of the composition.

Advantageously, the composition according to the invention comprises between 1 and 5% of stearic acid, preferably about 3% w/w compared to the total weight of the composition.

Advantageously, the composition according to the invention comprises between 0 and 2% of trolamine, preferably about 0.5% w/w compared to the total weight of the composition.

Appropriate consistency agents comprise glycerol monostearate and PEG.

Preferably, the composition according to the invention comprises about 5% of consistency agents w/w compared to the total weight of the composition.

Advantageously, the composition according to the invention comprises between 2 and 10% of glycerol monostearate, preferably about 5% w/w compared to the total weight of the composition.

Appropriate preservatives comprise methyl parahydroxybenzoate, propyl parahydroxybenzoate and chlorocresol.

Preferably, the composition according to the invention comprises about 0.1 % of preservatives w/w compared to the total weight of the composition.

Advantageously, the composition according to the invention comprises between 0 and 1% of methyl parahydroxybenzoate, preferably about 0.09% w/w compared to the total weight of the composition.

Advantageously, the composition according to the invention comprises between 0.05 and 1% of propyl parahydroxybenzoate, preferably about 0.1% w/w compared to the total weight of the composition.

Appropriate spreading agents comprise dimethicone and polydimethylcyclosiloxane.

Preferably, the composition according to the invention comprises about 2% of spreading agents w/w compared to the total weight of the composition.

Advantageously, the composition according to the invention comprises between 0.2 and 2% of dimethicone, preferably about 0.5% w/w compared to the total weight of the composition.

Advantageously, the composition according to the invention comprises between 1 and 3% of polydimethylcyclosiloxane, preferably about 2.5% w/w compared to the total weight of the composition.

Appropriate water-binding agents comprise polyethylene glycol, preferably polyethylene glycol 600.

Preferably, the composition according to the invention comprises about 8% of water-binding agents w/w compared to the total weight of the composition.

Advantageously, the composition according to the invention comprises between 2 and 10% of polyethylene glycol, preferably about 5% w/w compared to the total weight of the composition.

Water used in the aqueous phase of the emulsion can be distilled water or thermal water with dermato-cosmetic properties.

Advantageously, the composition according to the invention comprises :
- about 15 % of glycerol,
- about 8% of white soft paraffin,
- about 2% of liquid paraffin,
- about 1 at 5% of stearic acid,
- about 2 at 10% of glycerol monostearate,
- about 1 at 3% of polydimethylcyclosiloxane,
- about 0.2 at 2% of dimethicone,
- about 2 at 10% of polyethylene glycol 600,
- about 0 to 2% de trolamine,
- about 0.05 to 1% de propyl parahydroxybenzoate
- water q.s..

The invention will further be illustrated in view of the following figures and examples.

### FIGURES AND TABLES:

Figure 1: Corneometry - Mean % of evolution in relation to baseline values in Example 2.
Figure 2: TEWL - Mean % of evolution in relation to baseline values in Example 2.
Figure 3: Time-course severity score of uremic xerosis in the study population of Example 3 with Composition A applied twice daily for 56 days. * p<0.001 (intra-group analysis).
Figure 4: Time-course severity score of uremic pruritus in the study population of Example 3 under treatment with Composition A applied twice daily for 56 days. *p<0.0001 (intra-group analysis).
Figure 5: Time-course severity score of uremic xerosis in the study population of Example 4 under treatment with Composition B (up to 133 days) and vehicle (up to day 28 only), applied once daily for 133 days. * p < 0.05, ** p < 0.005 (intergroup analysis)
Table 1: Demographic features and health status of the study population of Example 3 at baseline.
Table 2: Comparison of the uremic xerosis severity on the lower legs between the two treatment groups after 7 days in the study population of Example 3.

### EXAMPLES

### Example 1 : Formulations

### Composition A

- 15 g of glycerol,
- 8 g of white soft paraffin
- 2 g of liquid paraffin,
- and as excipients stearic acid, glycerol monostearate, polydimethylcyclosiloxane, polyethylene glycol (PEG) 600, methyl parahydroxybenzoate, propyl parahydroxybenzoate, dimethicone, trolamine, Amerchol L101®,
- water q.s. 100 g.

### Composition B

- 15 g of glycerol,
- 8 g of white soft paraffin
- 2 g of liquid paraffin,
- and as excipients stearic acid, glycerol monostearate, polydimethylcyclosiloxane, polyethylene glycol (PEG) 600, propyl parahydroxybenzoate, dimethicone, trolamine,
- water q.s. 100 g.

### Example 2 : Study of the separate and combined contribution of the active ingredients glycerol and paraffin in the Example 1 formulation to the restoration of skin hydration and barrier function

A total of 55 healthy volunteers (age range: 18-40 years) with a common xerosis of mild to moderate severity (Kligman's score: 2 or 3) were enrolled in a comparative, randomised, double-blind study. Glycerol alone (15%), paraffin alone (10%), their combination (Composition A) and their vehicle were tested in each individual as a single skin application in a thin layer (1 finger tip unit, i.e. corresponding to about *1 gr* of product) on their randomly allocated sites of the forearms (2 test treatments by forearm). Randomisation to test sites was performed using a Latin square procedure. Skin water content was measured by corneometry using the CM 825 apparatus (Courage and Khazaka) and skin barrier function was evaluated by TEWL, using evaporimetry (Courage and Khazaka), up to 12 hr after the test treatment application (baseline, 1 hr, 2 hr, 4 hr, 8 hr, 10 hr and 12 hr). Instrumental measurements were made in controlled environmental conditions (temperature: 22 ± 2°C; relative humidity: 50 ± 10%). The local tolerance of the test products was evaluated by a dermatologist at the end of the study (12 hr). The intended analysis plan was an analysis of variance in cross-over.

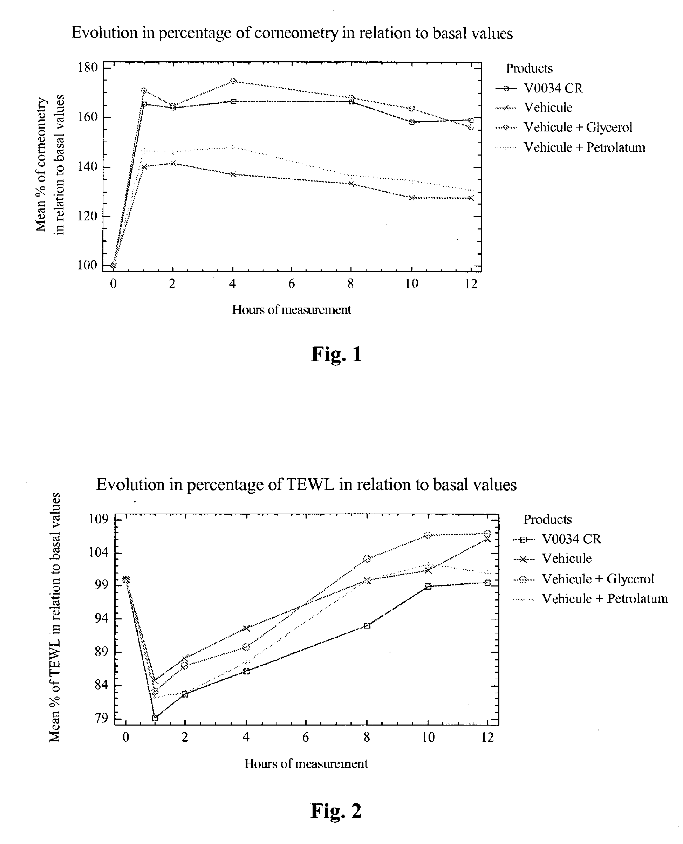

Among the 55 randomised subjects, 4 subjects were not included in the statistical analysis for major protocol violations. Thus, the population studied for efficacy comprised 51 volunteers (all-patients-treated or APT population). After opening the randomisation code, two volunteers were found to have aberrant TEWL values, which were considered related to technical problems. Instead, differences between treatments were determined by the analysis of variance (ANOVA) with repeated measures, adjusted by the two following multiple pair wise comparison tests: the Bonferroni *test* and the Dunnett test. For completion, results in both APT populations (restricted APT *population* of 49 volunteers, full APT population of 51 patients) are presented below. Results on skin water content in the restricted APT population are illustrated in **Figure 1****.** Comparison of baseline values showed no difference between treatment groups in both populations (restricted APT, full APT). In the restricted APT population analysis, treatment with vehicle alone resulted in an increase in SC hydration by about 40% after 1 hr, that progressively decreased up to 12 hr. Paraffin had a mild hydrating effect compared to vehicle, although significant (p<0.03). Conversely, glycerol induced a dramatic increase in water content by 65% compared to baseline, and this increase was maintained all over the observation period (12 hr). Difference between the glycerol treatment group and the vehicle group was statistically different (p<0.0001, Bonferroni test). Similarly, the combination glycerol and paraffin resulted in a high skin hydration that was comparable to glycerol alone (p<0.0001 compared to vehicle, Bonferroni test; confirmed by Dunnett test: p=0.0001). In the full APT population analysis, results and statistical data were similar to those obtained in the restricted APT population analysis, except for paraffin, which did not show significant hydrating effect compared to vehicle. Accordingly, the hydration capacity of the Composition A was mainly attributed to glycerol.

Results on TEWL in the restricted APT population are illustrated in **Figure 2****.** TEWL values between treatment groups were similar at baseline in both APT populations. In the restricted APT population analysis, vehicle alone induced a decrease in TEWL up to 8 hr, that was maximum (15% reduction) after 1 hr. Glycerol alone did not significantly modify TEWL compared to vehicle. Paraffin alone was slightly but significantly more efficient than vehicle on TEWL reduction (p<0.02, Bonferroni test). Only the combination glycerol and paraffin resulted in a higher (about 20% reduction from baseline after 1 hr) and more lasting (up to 10 hr) decrease in TEWL, compared to vehicle (p<0.002, Bonferroni test; confirmed by Dunnett test: p=0.0009). In the full APT analysis, results and statistical data were comparable to the restricted APT population analysis, except that paraffin alone showed no significant effect compared to vehicle.

In conclusion, the occlusive capacity of Composition A was mainly exerted by the combination of glycerol and paraffin, whereas each active compound alone provided no (glycerol) or limited (paraffin) effects. Thus, the adjunction of glycerol to paraffin increases the occlusive effects of paraffin.

It was concluded that the proposed medicinal product had both a significant hydrating effect and an occlusive effect.

Glycerol alone showed a hydrating effect but no action on TEWL, and paraffin alone showed a limited effect on TEWL but no effect on skin hydration.

Only the combination of the two compounds exerted at best both hydrating and occlusive effects, indicating a synergistic action between glycerol and paraffin.

### Example 3: First clinical study

### Study population

One hundred (100) patients were enrolled in 4 participating centres (25/centre) according to the following inclusion criteria. The study population was to include male and female patients of at least 10 years of age, undergoing MRD (haemodialysis or peritoneal dialysis) due to end-stage renal disease (ESRD). These patients presented with uremic xerosis related to their ESRD status. Uremic xerosis was moderate to severe, i.e. with a severity score of 2 to 4, and spread onto two symmetric areas of the lower legs of equal severity. Uremic xerosis was associated or not with uremic pruritus.

Patients with a known allergy to one of the test ingredients, and those having an intercurrent condition which might have interfered with a good conduct of the study were excluded. Patients treated with any moisturizing or emollient topical preparation within 7 days prior to study entry, with an instable dosage schedule of antipruritics (e.g. antihistamines, cholestyramine, opioid inhibitors, charcoal) within 4 weeks prior to study entry, and patients with phototherapy within 8 weeks prior to study entry were also excluded.

No paediatric patient was finally included, as the participating centres were referral centres for adults only. Upon request of the local ethics committee, children were excluded from the enrolled population in one centre (France).

One randomised patient withdrew secondarily his consent the day after baseline (day 1) and never applied the test products. Accordingly, this patient was excluded from the analysis, and 99 randomised patients (53 males, 46 females, with a median age of 65 years) who received at least one application of the test products, were included in the intent-to-treat population.

The demographic features (age and gender) of the ITT study population are presented in **Table 1.**

**Table 1 - Demographic features and health status of the study population at baseline.**

| **Variables** | **N = 99** |
|---|---|
| Age (y, mean ± SEM) | 63.16 ± 1.28 |
| Gender (N, %): | |
| - Male | 53 (54%) |
| - Female | 46 (46%) |
| End Stage Renal Disease underlying disease (N, %): | |
| - Glomenular diseases | 25 (25%) |
| - Diabetic nephropathy | 15 (15%) |
| - Nephrosclerosis | 15 (15%) |
| - Polycystic kidney | 14 (14%) |
| - Congenital nephropathies | 1 (1%) |
| - Pyelonephritis | 5 (5%) |
| - Hypertensive nephropathy | 7 (7%) |
| - Others | 9 (9%) |
| - Unknown | 10(10%) |
| Type of MRD (N, %): | |
| - Haemodialysis | 90 (91%) |
| - Peritoneal dialysis | 9 (9%) |
| Duration of MRD (y, mean ± SEM) | 7.91 ± 0.71 |
| Duration of xerosis (y, mean ± SEM) | 5.4 ± 0.53 |
| Clinical severity of xerosis (N, %): | |
| - mild | 2 (2%) |
| - moderate | 50 (51%) |
| - severe to very severe | 47 (47%) |
| Total clinical score of xerosis (mean ± SEM) | 8.3 ± 0.3 |
| Association with pruritus (N, %): | |
| - Yes | 74 (75%) |
| - No | 25 (25%) |
| Severity of pruritus (mm VAS, mean ± SEM) | 40.64 ± 3.36 |
| Quality of life scores (mean ± SEM): | |
| - PCS | 30.51 ± 1.21 |
| - MCS | 41.29 ± 1.24 |
| - DLQI | 5.61 ± 0.57 |

### Study design

The study was multicentric and organised into 2 subsequent phases. Phase I (baseline to day 7) was a comparative period evaluating the test product (Composition A, active product) versus its vehicle intra-individually (left lower leg vs. right lower leg comparison). The test-product vehicle was the excipiendiary formula of Composition A devoid of the active ingredients, which served as a comparative hydrating control. It matched in color and appearance with Composition A. This phase was randomised and double-blind. Phase II (days 7 to 56) was a non-comparative open-labelled period evaluating the test product (Composition A) alone.

In phase I, patients applied twice daily each Composition A and the vehicle onto one randomly assigned lower leg for 7 days. The other xerotic areas of the body were asked not to be treated. In phase II, Composition A was applied twice daily onto all xerotic and pruritic areas of the body for 49 days. The amount of products to apply depended on the surface area of the lesions (1mg/cm²) and patients were informed about the dose to use for each treated areas in terms of finger tips, one finger tip corresponding approximately to 1 g of product. For optimising the amount of product to apply, a table recommending the number of finger tips of product on each body area was given to the patients.

Study visits were performed at baseline, day 7, day 28 and day 56. To minimise inter-observer variation in end-point measurements, each patient was evaluated by the same physician at all visits.

### Evaluation parameters

All the evaluations were blinded to treatment allocation. Response to treatment was defined as a decrease of at least 2 grades of the El Gammal' score on the lower leg at the end of Period I (day 7). In order to minimize variability between centers, a photograder illustrating each grade was provided. The other evaluation parameters of xerosis included a test-side preference made by the investigators on day 7 using a 3-point categorical scale (0=right side comparable to left side; 1=right side better than left side; 2= left side better than right side), and the instrumental measurements of scaling on each lower leg at baseline and day 7 using the D-Squame^{®} technique (SURFT parameter, which measures the extent and density of the scales; MOD, which measures the thickness of the scales). The time-course severity of uremic xerosis was assessed all over the study on 4 test sites (each lower leg, forearm without the arteriovenous shunt and the chest), and expressed as a total score. Patient-orientated evaluation comprised the autoascertainment of global pruritus at baseline, day 28 and day 56, using a 100-mm Visual Analogue Scale (VAS), and the assessment of their quality of life at baseline and day 56, using both the generic scale Short Form-12 (SF-12) questionnaire and the dermatology-specific Dermatology Life Quality Index (DLQI). Briefly, SF-12 is a multipurpose measure of general health status which dichomises a physical component (Physical Component Summary or PCS-12; PCS=50 in the overall population) and a mental component (Mental Component Summary or MCS-12; normal MCS=50), whereas DLQI measures the impact of dermatological diseases on patient life quality (DLQI < 0.5 in the population with healthy skin). Global tolerance by the investigators and product acceptability by the patients on efficacy, local tolerance and cosmetics were also assessed at study end. Adverse events were recorded all over the study.

### Data analysis

Statistical analysis of the primary efficacy parameter was made with the McNemar's test. The secondary efficacy qualitative parameters were evaluated by the Wilcoxon signed rank test or the sign test for the test side preference, and the quantitative parameters by the non parametric Wilcoxon test. A descriptive analysis was given for tolerance and adverse events.

### Results

Comparative data between treatment groups on day 7 are summarized in **Table 2.** Treatment response was observed in 72 patients (73%) on the leg treated by the active product, and in 44 patients (44%) on the leg treated by the vehicle. Difference between treatment groups was statistically significant (p<0.0001). Similarly, test side preference by the investigators was in favour of the active treatment (60 patients, 61%), whereas the vehicle treatment was found to be better in 13 patients (13%) and comparable to active in 26 patients (26%; p<0.0001). Scaling measurements by D-Squame^{®} on day 7 confirmed that the active product was efficient by reducing from baseline the extend and density of the scales (SURFT parameter) as well as their thickness (MOD parameter), in a statistically significant manner compared to vehicle (p< 0.0001).

**Table 2 - Comparison of the uremic xerosis severity on the lower legs between the two treatment groups after 7 days (Period I) in the study population.**

| **Study parameters** | **Active** (N = 99) | **Vehicle** (N = 99) | **P-value** |
|---|---|---|---|
| Treatment response (N, %): | | | |
| - Yes | 72 (73%) | 44 (44%) | < 0.0001 |
| - No | 27 (27%) | 55 (56%) | |
| Instrumental severity of xerosis (arbitrary units, mean ± SEM): | | | |
| - SURFT | | | |
| Baseline | 11.37 ± 0.64 | 10.25 ± 0.55 | |
| Day 7 | 3.19 ± 0.42 | 5.35 ± 0.57 | < 0.0001 |
| - MOD | | | |
| Baseline | 19.20 ± 0.98 | 18.02 ± 0.76 | |
| Day 7 | 7.83 ± 0.60 | 11.56 ± 0.93 | < 0.0001 |
| Test side preference: | | | |
| - Clearly better | 60 | 13 | < 0.0001 |
| - Comparable | ← 26 → | | |

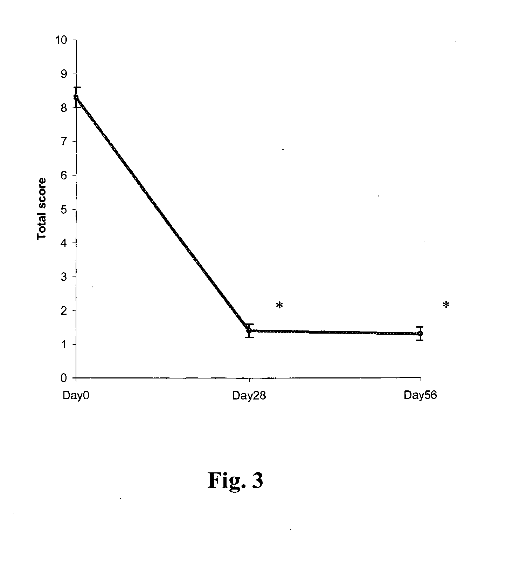

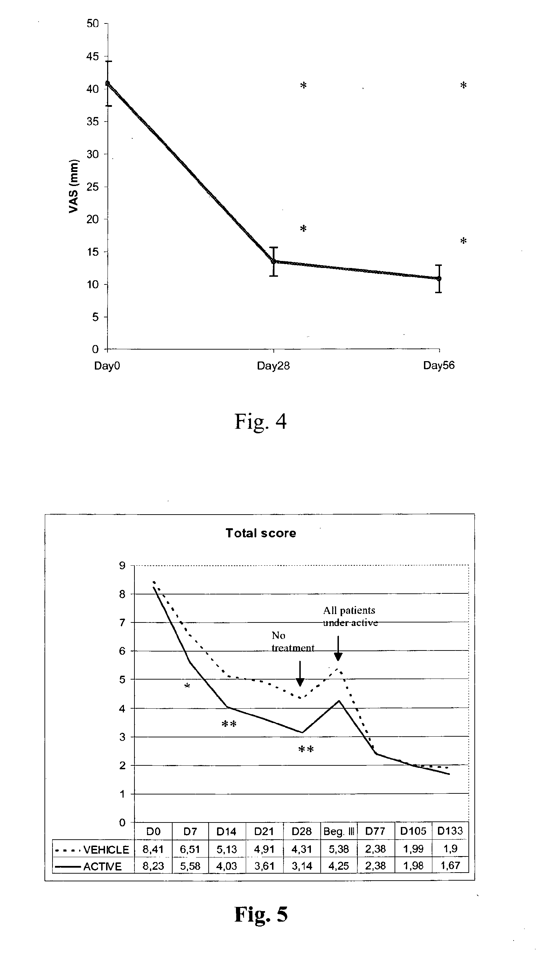

The time course of the total clinical score on the 4 test areas showed a dramatic decrease on day 28 (mean ± SEM: 1.4 ± 0.2), that was maintained on day 56 (1.3 ± 0.2 ; p<0.001 at the two visits, intra-group analysis; **Figure 3****)**. The overall pruritus as assessed by the patients (100-mm VAS) was also markedly amended on day 28 (mean ± SEM: 13.39 ± 2.19 mm) and day 56 (10.66 ± 2.14 mm; p<0.0001, intra-group analysis), i.e. by about 75% (Figure 4). On day 56, improvement of uremic xerosis and pruritus was associated with a marked improvement of the DLQI (mean ± SEM: 2.27 ± 0.46), and with a moderate improvement of the SF-12 (PCS: 33.89 ± 1.34; MCS: 46.72 ± 1.28). Difference between baseline scores and day 56 scores were statistically significant for all life quality scales (p<0.0001, intra-group analysis).

At study end (day 56), global tolerance of the test product by the investigators (N=93, missing data in 6 patients) was judged as very good in 82 patients (88%), good in 7 patients (8%), poor in 1 patient (1%) and very poor in 3 patients (3%). Product acceptability by the patients met high or very high satisfaction as regards to efficacy (82 out of 98 patients, 84%), local tolerance (84 out of 90 patients, 93%) and cosmetic acceptance (82 out of 88 patients, 93%). A total of twenty-one (21) adverse events were reported, among which 5 of them were considered to be related to the test product, occurring in 5 patients (5%). They all were local and included 2 exacerbations of pruritus, 2 local burning or hyperesthesia, and one irritative reaction (erythema). Four (4) adverse events were judged of mild intensity and 1 was severe. Finally, all but two were recorded to be resolved completely by discontinuating temporarily or definitely the product. One case was still unresolved at study end and another case had no follow-up record.

### Overall conclusion

This clinical study demonstrates that the topical Composition A, which combines glycerol and paraffin (solid and liquid form), is an effective treatment of uremic xerosis of moderate to severe intensity. Its main effect on xerotic lesions was achieved after 7 days, further improved after 28 days, and maintained at day 56. The Composition A effect was clinical, as observed by significant reduction of the clinical grading of the lesions, as well as instrumental (D-Squame®), as shown by the significant decrease of two parameters assessing the extension (SURFT) and the thickness (MOD) of scaling.

Treatment of uremic xerosis by the Composition A was associated with a marked improvement of uremic pruritus. This demonstrates that Composition A is able to amend uremic pruritus. It was also associated with a significant improvement of the quality of life.

The acceptability of Composition A by the patients as regards to efficacy, tolerance and ease of use was satisfactory to very satisfactory in the vast majority of patients. The local tolerance profile of the product was very good. Test-product related local AE included erythema (1%), pruritus (2%), local burning (1%) and algia (1%). No systemic AE was reported to be probably related to test product.

In conclusion, Composition A showed a good efficacy and safety profile in patients with moderate to severe uremic xerosis, as well as in uremic pruritus.

### Example 4: Second clinical study: Long-term efficacy and safety of Composition B in patients with moderate-to-severe uremic xerosis

The objectives of the study were the following: 1°) to demonstrate the long-term efficacy of Composition B on uremic xerosis in the real life setting (initial treatment, maintenance treatment); 2°) to assess the long-term local tolerance and patient acceptability of Composition B after long-term use; and 3°) to determine the dosage regimen of Composition B at therapeutic doses in a long-term treatment course in uremic xerosis patients.

### Study population

Enrolled patients were patients with moderate to severe uremic xerosis, with the same inclusion and exclusion criteria as those enrolled in the study of Example 3. Patients with maintenance renal dialysis (MRD, haemodialysis, peritoneal dialysis) of both sexes, of at least 18 years of age, suffering from uremic xerosis with a severity score of at least 2, on at least one of the 5 tests areas (right lower leg, left lower leg, forearm with no arterio-venous shunt, chest, dorsum of the neck) were enrolled.

Two hundred and forty (240) patients were planned; 237 were selected and 235 were finally randomized in the study, i.e. 118 patients in the active group (Composition B) and 117 patients in the vehicle group.

### Study design

The study comprised the 3 following periods:
Period I when the study was randomised, vehicle-controlled, double-blind, two-parallel group, and comparative (initial treatment period).
Period II, that was randomised, double blind, two-parallel group and comparative, with patients receiving no treatment (treatment-free follow-up).
And Period III, that was open-labelled, non controlled, in which all patients applied the active product only, maintenance treatment period.

The active product was Composition B of Example 1. The reference product was its vehicle (i.e. Composition B without glycerol, white soft paraffin and liquid paraffin) used as comparative hydrating control. During Period I, Composition B or its vehicle was applied once daily for the topical application of 2 to 4 finger tips (according to localisation of xerotic areas to treat). During Period III, Composition B only was applied *ad libitum.*

The duration of treatment was:
- 28 days (from baseline to day 28) during Period I;

Up to 21 days (from day 28 up to day 49) during Period II. Only patients with non persisting lesions (i.e. patients with all test areas having a severity score of less than 2) at the end of Period I (day 28) entered Period II (and if relapse occurred on day 35 or on day 42, they entered Period III). Patients with persisting lesions (i.e. patients having at least one test area with a severity score of at least 2) on day 28 entered directly Period III. - At least 84 days (from day 49 or earlier if relapse during Period II occurred, to day 133) during Period III.

### Evaluation parameters and statistical analysis:

Evaluation parameters were performed on the Intent-To-Treat Population. The main efficacy parameters were as follows:
- Severity of xerosis: response rates in the two treatment groups were compared at the end of Period I (on day 28), using the Fisher exact test.
- Time to relapse between the two treatment groups were compared, using the Log-rank test

The secondary parameters were the following:
- Time-course severity score ofuremic xerosis (Wilcoxon signed-rank test),
- Scaling measurements (D-squame®, Wilcoxon signed-rank test),
- Acceptability of the test product by the patients on efficacy, local tolerance and ease of use (descriptive analysis),
- Frequency of application and product consumption (descriptive analysis),
- Adverse events (descriptive analysis).

### Efficacity results:

This pivotal phase III trial investigating the long-term efficacy and safety of a combination of glycerol (15%) and paraffin (10%) in patients with moderate-to-severe xerosis confirmed the previous efficacy results of the more short-term study of Example 3. The clinically significant effect of the Composition B was here demonstrated using a once a day treatment regimen for 4 weeks (Period I), that was comparable to the clinical effect of Composition A used twice daily for 1 week. Indeed, treatment response was demonstrated in 72 patients treated with Composition B (60%) and in 48 patients treated with the vehicle (41%). Difference in favor of Composition B was statistically significant (p = 0.004).

When active treatment was discontinued after initial therapy (Period II), a mild remanent effect of Composition B was observed, although not significant in comparison to the vehicle. This suggests that the active product has mainly a palliative effect that requires continuous therapy even when lesions remit.

The time-course study of the clinical score of uremic xerosis confirmed the rapid and dramatic effect of Composition B, that was statistically significant from I week onwards, compared to vehicle (Period I; **Figure 5****).** Treatment discontinuation resulted in a progressive recurrence of the lesions after 3 weeks (Period II). Finally, the treatment of all patients by Composition B resulted again in lesional remission with an average frequency of once daily application regimen (Period III). Durability of the effect was very good, with very low recurrence rate of the disease under maintenance therapy (2% at the end of Period III). Like in Study of Example 3, the instrumental measurement of scaling using D-Squame^{®} confirmed the clinical evaluation made by the investigators, with a statistically significant difference in the reduction of scales during Composition B treatment (p<0.001, intra-group analysis) and in comparison to vehicle (p<0.01, inter-group analysis).

Acceptability of Composition B by the patients was very high as regards to efficacy (satisfactory to very satisfactory in 92% of the patients) as well as ease-of-use (93%).

This phase III pivotal study could also establish the dose regimen for the product in the indication moderate-to-severe uremic xerosis, according to the following schedule: a long-term and continuous therapy at the dosage of one application per day, with an amount of about 10 gr of cream per application on all over the xerotic areas.

### Safety results:

The good safety profile of Composition B after its long-term use in patients with uremic xerosis was demonstrated. As topical product containing non systemically absorbed active ingredients, administration of the product did not result in any systemic side-effect. Like in study of Example 3, product related adverse events were infrequent , reported in 12/235 patients (5%). These AE were all local and comprised irritation or erythema reaction at application site (5 patients, 2.1%), allergic dermatitis or local vesicles (3 patients, 1.3%), exacerbated pruritus (3 patients, 1.3%) and dishydrosis (1 patient, 0.4%). These data should be highlighted as very favorable results, in the context of the target population who are known to be particularly prone to develop irritation to topicals in general.

Another demonstration of the excellent local tolerance profile was the patient acceptability of the product on local tolerance (good to very good tolerance in 96% of the patients), which was also in accordance with the results in Study of Example 3.

### References

1. Ponticelli C and Bencini PL. Dermatology disorders in Oxford textbook of clinical nephrology. Oxford university press: 1995-2002, 1998*.*
2. Southi P and Commens C. Pruritus in dialysis patients. Med J Austral. 146 : 397-398, 1987*.*
3. Morton CA, Lafferty M, Hau C, Henderson I, Jones M, Lowe JG. Pruritus and skin hydration during dialysis. Nephrol. Dial. Transplant. 11: 2031-2036, 1996*.*
4. Szepietowski JC, Reich A, Schwartz A. Uremic xerosis. Nephrol. Dial. Transplant. 19 : 1-4, 2004*.*
5. Tsutsumi H, Utsugi, T, Hayashi S. Study on the occlusivity of oil films. J. Soc. Csmet. Chem. 30: 345-356, 1979
6. Marti-Mestres G, Passet J, Maillols H, Van Sam V, Guilhou J.J., Mestres J.P., Guillot B. Evaluation expérimentale de l'hydratation et du pouvoir occlusive in vivo et in vitro d'excipients lipophiles et de leurs émulsions phase huile continue. International Journal of Cosmetic Science 16 : 161-170, 1994*.*
7. Bettinger J, Gloor M, Gehring W. Influence of a pretreatment with emulsions on the dehydration of the skin by surfactants. Int. J. Cosm. Sci. 16 : 53-60, 1994*.*
8. Grunewald AM, Gloor M, Gehring W, Kleesz P. Barrier creams - commercially available creams versus urea - and glycerol- containing oil- in-water emulsions. Dermatosen 43: 69-74, 1995*.*
9. Bettinger J, Gloor M, Peter C, Kleesz P, Fluhr J, Gehring W. Opposing effects of glycerol on the protective function of the horny layer against irritants and on the penetration of hexyl nicotinate. Dermatology 197: 18-24, 1998*.*
10. Fluhr JW, Gloor M, Lehmann L, Lazzerini S, Distante F, Berardesca E. Glycerol accelerates recovery of barrier function in vivo*. Acta. Derm. Venereol. 79: 418-421, 1999*.*
11. Sagiv ae, Dikstein s, Ingber A. The efficiency of humectants as skin moisturizers in the presence of oil. Skin Res. Tech. 7 : 32-35, 2001.
12. Barany E, Lindberg M, Loden M. Unexpected skin barrier influence from nonionic emulsifiers. Int. J. of Pharm. 195:189-195, 2000.

## Claims

1. A pharmaceutical composition comprising a combination of glycerol, white soft paraffin and liquid paraffin as active ingredients for topical use to treat uremic pruritus.

2. Composition for topical use to treat uremic pruritus according to claim 1, wherein said topical use has a once per day dosage regimen.

3. Composition for topical use to treat uremic pruritus according to 2, wherein said once per day dosage regimen is for at least 2 weeks, preferably 4 weeks.

4. Composition for topical use to treat uremic pruritus according to any of the preceding claims, comprising less than 15, preferably less than 10, components.

5. Composition for topical use to treat uremic pruritus according to any of the preceding claims, the white soft paraffin having a dropping point comprised between 35 and 70°C (The dropping point is measured according to the process 2.2.17 described in «European Pharmacopeia», 6th edition).

6. Composition for topical use to treat uremic pruritus according to claim 5, the white soft paraffin having a dropping point comprised between 51 and 57°C, preferably about 54°C (The dropping point is measured according to the process 2.2.17 described in «European Pharmacopeia», 6th edition).

7. Composition for topical use to treat uremic pruritus according to any of the preceding claims, the white soft paraffin having a consistency comprised between 175 and 195 1/10 mm (cone penetration at 25°C), preferably of about 185 1/10 mm (cone penetration at 25°C).

8. Composition for topical use to treat uremic pruritus according to any of the preceding claims, the white soft paraffin having a viscosity comprised between 4 and 5 cSt at 100°C preferably of about 4.8 cSt at 100°C.

9. Composition for topical use to treat uremic pruritus according to any of the preceding claims comprising about 15% of glycerol, about 8% of white soft paraffin and about 2% of liquid paraffin.

10. Composition for topical use to treat uremic pruritus according to any of the preceding claims comprising one or several excipients selected from the group consisting of stearic acid, glycerol monostcarate, polydimethylcyclosiloxane, dimethicone, polyethylene glycol 600, trolamine, propyl parahydroxybenzoate.

11. Composition for topical use to treat uremic pruritus according to any of the preceding claims in the form of an oil-in-water (O/W) emulsion.

## Patentansprüche

1. Pharmazeutische Zusammensetzung, die eine Kombination aus Glyzerin, Vaseline und flüssigem Paraffin als Wirkstoffe zur topischen Anwendung bei der Behandlung von urämischem Pruritus umfasst.

2. Zusammensetzung zur topischen Anwendung bei der Behandlung von urämischem Pruritus nach Anspruch 1, wobei das Dosierungsschema eine Einnahme von einmal am Tag vorsieht.

3. Zusammensetzung zur topischen Anwendung bei der Behandlung von urämischem Pruritus nach Anspruch 2, wobei sich das Dosierungsschema von einmal am Tag über mindestens 2 Wochen, vorzugsweise 4 Wochen erstreckt.

4. Zusammensetzung zur topischen Anwendung bei der Behandlung von urämischem Pruritus nach einem der vorhergehenden Ansprüche, die weniger als 15, vorzugsweise weniger als 10 Komponenten umfasst.

5. Zusammensetzung zur topischen Anwendung bei der Behandlung von urämischem Pruritus nach einem der vorhergehenden Ansprüche, wobei die Vaseline einen Tropfpunkt zwischen 35 und 70 °C aufweist (Der Tropfpunkt wird gemäß dem in "European Pharmacopoeia", 6. Ausgabe, beschriebenen Prozess 2.2.17 gemessen).

6. Zusammensetzung zur topischen Anwendung bei der Behandlung von urämischem Pruritus nach Anspruch 5, wobei die Vaseline einen Tropfpunkt zwischen 51 und 57 °C, vorzugsweise von ungefähr 54 °C aufweist (Der Tropfpunkt wird gemäß dem in "European Pharmacopoeia", 6. Ausgabe, beschriebenen Prozess 2.2.17 gemessen).

7. Zusammensetzung zur topischen Anwendung bei der Behandlung von urämischem Pruritus nach einem der vorhergehenden Ansprüche, wobei die Vaseline eine Konsistenz zwischen 175 und 195 1/10 mm (Konuspenetration bei 25 °C), vorzugsweise von ungefähr 185 1/10 mm (Konuspenetration bei 25 °C) aufweist.

8. Zusammensetzung zur topischen Anwendung bei der Behandlung von urämischem Pruritus nach einem der vorhergehenden Ansprüche, wobei die Vaseline eine Viskosität zwischen 4 und 5 cSt bei 100 °C, vorzugsweise von ungefähr 4,8 cSt bei 100 °C aufweist.

9. Zusammensetzung zur topischen Anwendung bei der Behandlung von urämischem Pruritus nach einem der vorhergehenden Ansprüche, die ungefähr 15% Glyzerin, ungefähr 8% Vaseline und ungefähr 2% flüssiges Paraffin umfasst.

10. Zusammensetzung zur topischen Anwendung bei der Behandlung von urämischem Pruritus nach einem der vorhergehenden Ansprüche, die einen oder mehrere aus der Gruppe bestehend aus Stearinsäure, Glyzerinmonostearat, Polydimethylcyclosiloxan, Dimeticon, Polyethylenglykol 600, Trolamin, Propyl-Parahydroxybenzoat ausgewählte Hilfsstoffe umfasst.

11. Zusammensetzung zur topischen Anwendung bei der Behandlung von urämischem Pruritus nach einem der vorhergehenden Ansprüche, in der Form einer Öl-in-Wasser-Emulsion (O/W).

## Revendications

1. Composition pharmaceutique comprenant une combinaison de glycérol, de vaseline et de paraffine liquide en tant que principes actifs pour une utilisation topique pour traiter le prurit urémique.

2. Composition pour une utilisation topique pour traiter le prurit urémique selon la revendication 1, où ladite utilisation topique suit un régime posologique d'une fois par jour.

3. Composition pour une utilisation topique pour traiter le prurit urémique selon la revendication 2, où ledit régime posologique d'une fois par jour est suivi pendant au moins 2 semaines, de préférence 4 semaines.

4. Composition pour une utilisation topique pour traiter le prurit urémique selon l'une quelconque des revendications précédentes, comprenant moins de 15, de préférence moins de 10, composants.

5. Composition pour une utilisation topique pour traiter le prurit urémique selon l'une quelconque des revendications précédentes, la vaseline ayant un point de goutte compris entre 35 et 70 °C (Le point de goutte est mesuré selon le procédé 2.2.17 décrit dans la « Pharmacopée Européenne », 6^{ème} édition).

6. Composition pour une utilisation topique pour traiter le prurit urémique selon la revendication 5, la vaseline ayant un point de goutte compris entre 51 et 57 °C, de préférence environ 54 °C (Le point de goutte est mesuré selon le procédé 2.2.17 décrit dans la « Pharmacopée Européenne », 6^{ème} édition).

7. Composition pour une utilisation topique pour traiter le prurit urémique selon l'une quelconque des revendications précédentes, la vaseline ayant une consistance comprise entre 175 et 195 1/10 mm (pénétration du cône à 25 °C), de préférence d'environ 185 1/10 mm (pénétration du cône à 25 °C).

8. Composition pour une utilisation topique pour traiter le prurit urémique selon l'une quelconque des revendications précédentes, la vaseline ayant une viscosité comprise entre 4 et 5 cSt à 100 °C, de préférence d'environ 4,8 cSt à 100 °C.

9. Composition pour une utilisation topique pour traiter le prurit urémique selon l'une quelconque des revendications précédentes, comprenant environ 15 % de glycérol, environ 8 % de vaseline et environ 2 % de paraffine liquide.

10. Composition pour une utilisation topique pour traiter le prurit urémique selon l'une quelconque des revendications précédentes, comprenant un ou plusieurs excipients choisis dans le groupe constitué d'acide stéarique, monostéarate de glycérol, polydiméthyl-cyclosiloxane, diméthicone, polyéthylène glycol 600, trolamine, parahydroxybenzoate de propyle.

11. Composition pour une utilisation topique pour traiter le prurit urémique selon l'une quelconque des revendications précédentes, sous la forme d'une émulsion huile dans l'eau (H/E).
